## Europäisches Patentamt
## European Patent Office
## Office européen des brevets

(11) Publication number: **0 094 232**
A2

## EUROPEAN PATENT APPLICATION

(21) Application number: **83302599.2**

(22) Date of filing: **09.05.83**

(51) Int. Cl.³: **A 61 J 3/00**, A 61 M 31/00

(30) Priority: **11.05.82 US 377163**

(43) Date of publication of application: **16.11.83**
**Bulletin 83/46**

(84) Designated Contracting States: **CH DE FR GB IT LI NL SE**

(71) Applicant: **Detrano, Salvatore J., 65 Sterling Avenue, Weehawken New Jersey 07087 (US)**

(72) Inventor: **Detrano, Salvatore J., 65 Sterling Avenue, Weehawken New Jersey 07087 (US)**

(74) Representative: **Froud, Clive et al, Elkington and Fife High Holborn House 52/54 High Holborn, London WC1V 6SH (GB)**

(54) **Anal suppository.**

(57) An anal suppository characterised in that it is capable of being inserted and retained in the anal canal and melting or dissolving therein while dispersing medication to the walls of the anal canal, the suppository comprising a composition which melts or dissolves in the anal canal and having a configuration which comprises an enlarged proximal tip (12) extending from one end of a cylindrically-shaped axial shaft (16) and an enlarged distal portion (22) extending from the opposite end of the axial shaft wherein a flexible communicating means (30) composed of a flexible and atraumatic composition, relatively inert to the environment of the anal canal is embedded in the central core of the suppository from the proximal tip through the axial shaft to the distal portion and anchored in the proximal and distal portions is disclosed.

## "ANAL SUPPOSITORY"

This invention relates to an anal suppository; more particularly, it relates to a dissolvable anal suppository which may be used in a method for treating diseases of the anus by providing prolonged contact of a drug or other therapeutic substance with the region of the anal canal.

The anus is the terminal portion of the intestinal tract and is located below the rectum which is the reservoir for intestinal residue. The anus is normally closed except to permit expulsion of excreta. Pain suffered in the terminal portion of the intestine normally originates from the anus since the entire length of the anal canal is endowed with an abundance of sensory nerve fibres. The rectum, on the other hand, is almost devoid of sensory nerves, except for the sensation of fullness. The most common ailments of the anus include internal and external hemorrhoids, proctitis sphinctericus, cryptitis, anusitis, fistulae, fissures, abscesses, traumatic lesions, warts or condyloma, venereal disease, healing post-operative wounds, itch, blood clots, psycogenic and psychosomatic complaints and cancer. Every one of the above pathologies causes severe anal pain and discomfort.

Various topical medications are known for promoting healing and for relieving the discomfort caused by diseases of the anus. However, the location of the ailment within the anal canal and the complex physiology of the anorectal region presents several unique problems in terms of physically distributing medication to the injured area and for maintaining a suitable concentration of the medication for the required treatment time.

A simple insertion of medication into the anal canal is generally inadequate and is an unsuccessful method of therapy because the medication is too quickly dissipated from the treated area. Such insertion is also aesthetically and hygienically undesirable. The most popular method of applying medication within the anal canal is from a transportive medium capable of dispensing the medication uniformly to the region of the anal canal for a predetermined period of time. There are two general types of transportive media known in the art.

The first type of transportive medium is designed to dissolve or melt while in the anal canal and to disperse the medication during the melting period. Such dissolvable suppositories are typically of a cylindrical or bullet shape, although other shapes have been suggested. The second type of transportive medium is a solid, removable device which contains the drug or therapeutic substance. This device has perforations to allow the medication within to escape and be transmitted to the tissue of the anal canal. Such removable devices may function, for example, as a suppository holder where a dissolvable suppository held within melts from body heat, dissolving slowly to disperse the medicine through the holder to the anal canal. The known devices of these types, however, are unsuccessful in uniformly distributing the medication and maintaining contact with the injured area of the anal canal for the time required for treatment and for dispersion of all of the medication to the injured area.

A primary cause of the failure of prior anal medicating devices is the unique physiology of the anorectal region. For example, the muscles of the anal canal, the sphincter muscles, cause the anal canal to expel objects within the canal, either out of the body or into the rectum. The muscular forces tending to move objects upward in the anal canal toward the rectum are referred to as cephalad forces, while the muscular forces tending to expel objects downward and out of the body are referred to as caudad forces. Treatment by known suppositories is unsuccessful because, for example, the caudad and/or cephalad forces cause the suppository to be expelled from the anal canal prior to the desired treatment time and complete dispersal of the medication. Therefore, one objective of an improved suppository is to improve its resistance to the natural expulsion by the muscles in the anorectal region and to extend its residence time in the anal canal to deliver medication uniformly to the injured tissue.

One attempted solution to the problem of maintaining a suppository in the anal canal is described in U.S. Patent No. 604,063. According to this disclosure, a dissolvable suppository is attached by an integral shank to a solid anchor. When the suppository is inserted into position in the anus, the anchor remains outside the body. The short length of the described suppository and its narrow proximal end means that it will not extend to the rectal end of the anus and sufficiently disperse medication to this region. The anchor provides an obstacle to the upward (cephalad) force created by the sphincter muscles of the anal canal and prevents the suppository from being propelled into the rectum. However, this suppository does not have any means to oppose the downward caudad force and thereby its slipping downward and ultimately being expelled out of the body. One reason for the expected slippage downward or expulsion of this suppository is the natural vermicular action of the anal sphincter muscles.

Another suppository comprised of a dissolvable composition is described in U.S. Patent No. 3,126,887. The body of this suppository comprises an enlarged head, a stem portion and an enlarged tail portion. The enlarged head lodges at the upward end of the anal canal and is designed to resist downward (caudad) expulsion and the enlarged tail is designed to resist the upward (cephalad) force. As will be discussed below, this suppository does not achieve the objective of prolonged contact with the anal canal because the suppository tends to break by action of the sphincter muscles and the pieces of the suppository are expelled in both directions. Another dissolvable suppository of this type is shown in U.S. Patent No. 3,840,010.

Devices that are solid, non-dissolvable and must be removed from the anal canal after use are described in U.S. Patent Nos. 823,499; 3,894,539 and 3,537,454. Each of these devices have rigid outer shells into which a dissolvable suppository is inserted. As the suppository within melts, the medicine is expected to migrate to the anal canal through perforations in the suppository shell. One problem with such devices is that the anal canal flexes up or tightens against the outer surface of the rigid shell of the device blocking the perforations therein and preventing uniform distribution of the medication. Although some of these devices may tend to remain within the anal canal during the desired treatment time, these devices do not attain uniform distribution of medicine within the anal canal and require careful removal and tend to be traumatic and/or uncomfortable to the patient in use. These devices are also more expensive than dissolvable suppositories, particularly for the occasional user.

The present invention relates to a dissolvable suppository for providing medicinal therapy to the anal canal, the suppository providing a residence time and rate of dissolution within the anal canal of sufficient duration to provide the desired application of medicine.

The present invention provides an anal suppository characterised in that it is capable of being inserted and retained in the anal canal and melting or dissolving therein while dispersing medication to the walls of the anal canal, the suppository comprising a composition which melts or dissolves in the anal canal and having a configuration which comprises an enlarged proximal tip (12) extending from one end of a cylindrically-shaped axial shaft (16) and an enlarged distal portion (22) extending from the opposite end of the axial shaft wherein a flexible communicating means (30) composed of a flexible and atraumatic composition, relatively inert to the environment of the anal canal is embedded in the central core of the suppository from the proximal tip through the axial shaft to the distal portion and anchored in the proximal and distal portions.

In a preferred embodiment, the present suppository is a body of dissolvable composition comprising a bullet-shaped proximal tip extending from one end of a cylindrically shaped axial shaft and a mushroom-shaped distal portion extending from the other end of the axial shaft with a non-dissolvable, atraumatic, pharmacologically-acceptable, flexible communicating means, such as a filament, string or cord, embedded within the body and running from the proximal tip through the centre core of the axial shaft to the distal portion, the communicating means being anchored in the proximal tip and in the distal portion. The communicating means may contain enlargements at its ends, such as tabs on an extruded filament or knots on a cord or string, to provide means for anchoring. The filament may extend through the distal surface to provide a tail for ease of handling. By a "dissolvable composition" is meant a base composition which is sufficiently hard at room temperature to allow for handling, packaging and insertion, but which melts or dissolves in the environment of the anal canal. The dissolvable composition may be a therapeutic material or a vehicle or carrier for an active therapeutic ingredient contained therein.

In use, the proximal tip of the suppository is positioned at the junction of the rectum and anus known as the ano-rectal, dentate or pectinate line. In this position, the proximal tip provides resistance to the downward (caudad) expulsive force exerted by the sphincter muscles of the anus. The axial shaft occupies the anal canal and comes into direct contact with the full length thereof. The distal end is positioned partly within the anal canal and partly outside the anal verge to provide resistance to the upward (cephalad) force exerted by the sphincter muscles surrounding the anal canal.

The dimensions of the suppository should be sufficient to enable the suppository to be situated in the anal canal as aforesaid. The actual shape and dimensions of the proximal tip and distal end may vary so long as the cross-sectional dimensions thereof are greater than that of the axial shaft and the shape and size thereof are sufficient to resist the action of the sphincter muscles yet be comfortable to the user. For example, the proximal and distal portions may have the same shape providing a symmetrical structure as in a dumbbell shape. However, the minimum diameter of the distal end is greater than the minimum diameter for the proximal tip.

By means of the present invention there is provided a method for providing a uniform distribution of medicine to the anal canal for a prolonged and predetermined period of time. This method may be used to treat diseases, such as anusitis, pruritis, fistulas and warts, of the anal canal; for example internal hemorrhoids, proctitis, sphinctericus, warts and trauma of the ano-rectal area; for example fissures, fistulas, warts and pruritis of the external anal orifice; for example pruritis ani, warts, fungus infection, allergies, fistulae, external hemorrhoids and thrombae of the perianal area; and post-hemorrhoidectomy (surgical) wounds of all of the aforementioned areas.

Referring to the accompanying illustrative drawings:

FIG. 1 is a plan view of a suppository constructed in accordance with the present invention;

FIG. 2 is a plan view of a suppository constructed in accordance with the present invention shown in the anal canal; and

FIGS. 3 and 4 are plan views of the anal canal showing the reflexive muscular action of the anal sphincter muscles.

FIG. 5 comprises three plan views of the anal canal. Views A and B show the canal containing a foreign body and View C shows the foreign body as it is expelled. The views show the vermicular action of the anal sphincter muscles forcing the foreign body out.

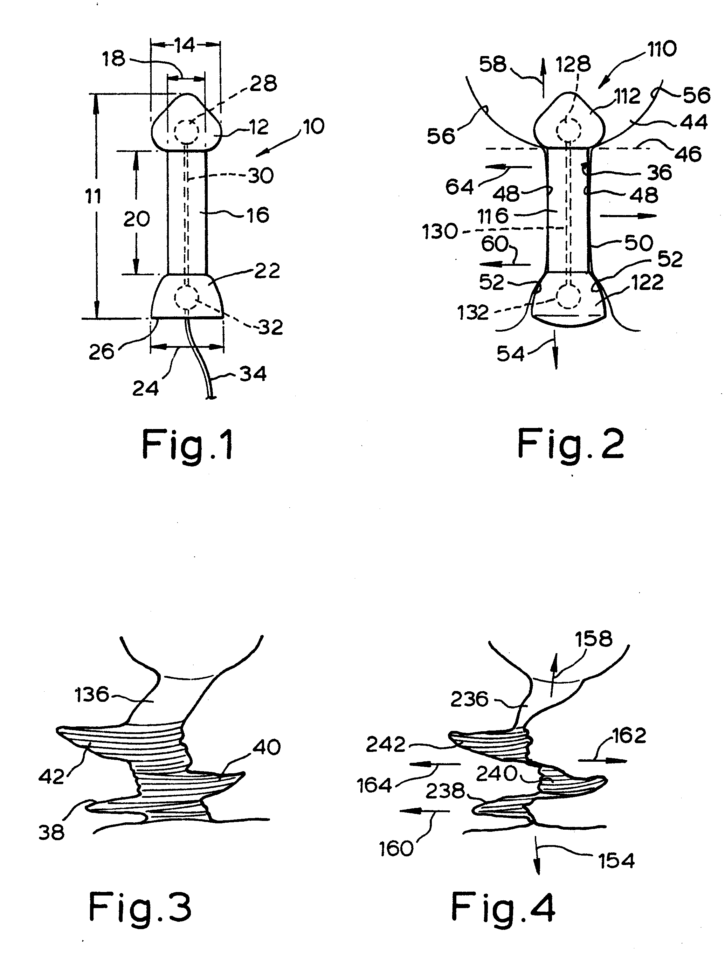

Referring to FIG. 1, suppository 10 comprises a proximal tip 12 extending from one end of axial shaft 16 and a distal end 22 extending from the other end of axial shaft 16. In this example, suppository 10 has an overall length 11 of 5.1 cm. The bullet-shaped proximal tip 12 has an approximate diameter 14 of 13 mm in this case. For example, axial shaft 16 has an approximate diameter 18 of 9 mm and an approximate length 20 of 28 mm. In this illustration, the diameter of distal end 22 gradually increases from 9 mm at its juncture with shaft 16 to a maximum diameter 24 of 17 mm at distal surface 26. Flexible communicating means 30 embedded within suppository 10 may be a two-ply twisted cord or string of about 2 mm diameter which runs axially from the proximal tip 12 through the shaft 16 into distal end 22. The flexible communicating means 30 has an enlarged end 28 in the proximal tip 12 and an enlarged end 32 in distal end 22. At distal surface 26, flexible communicating means 30 connects with optional retrieving line 34.

Suppository 10 is composed of traditional suppository materials, being a firm substance at room temperature and having a melting point at or below body temperature, which may be used to carry the appropriate medication. Flexible communicating means 30 is composed of an atraumatic, flexible material inert in the environment of the anal canal.

A preferred process for producing the suppository 10 involves pouring liquified suppository material containing medication into a mould, simultaneously embedding within the suppository 10 flexible communicating means 30 with enlarged ends 28 and 32.

Flexible communicating means 30 must be pharmacologically-acceptable and may simply consist of a length of string and enlarged ends 28 and 32 may simply be knots therein. Alternatively, flexible communicating means 30 may be a plastic filament and enlarged ends 28 and 32 may be tabs or integral beads of such plastic. The cross-section or diameter of the flexible communicating means must be sufficiently small so that the flexible communicating means is imperceptible to the sphincter muscles when it is exposed as a result of the melting or breaking of the axial shaft. Therefore, if the axial shaft should break or become dissolved prior to complete treatment by the dissolution of the proximal and distal portions, the remaining communicating means does not stimulate the reflex actions of the sphincter muscles enabling the muscles to return to a relaxed state.

FIGS. 3 and 4 illustrate relaxed and reflexed or contracted positions of the anal sphincter muscles and particularly the cutting or scissor effect. Normally, when the sphincter muscles are relaxed, the anal canal is closed. If the anal canal is forced open by an entering foreign body, the sphincter muscles remain relaxed only momentarily. The muscles are stimulated by the presence of the foreign body and immediately flex or contract. FIG. 3 shows the anal canal 136 opened with the three sphincter muscles profundus 42, superficialis 40 and subcutaneous 38 relaxed prior to reflex. In FIG. 4 the anal canal 236 is closed as a result of the contraction or flexing of the sphincter muscles. Sphincter superficialis 240 reflexively creates lateral force 162 in one direction, while sphincter profundus 242 and sphincter subcutaneous 238 create forces 164 and 160 in the opposite direction. These lateral forces constrict anal canal 236, causing expulsive downward (caudad) force 154 and expulsive upward (cephalad) force 158. These lateral forces cause a scissor or cutting action on any foreign body within the anal canal.

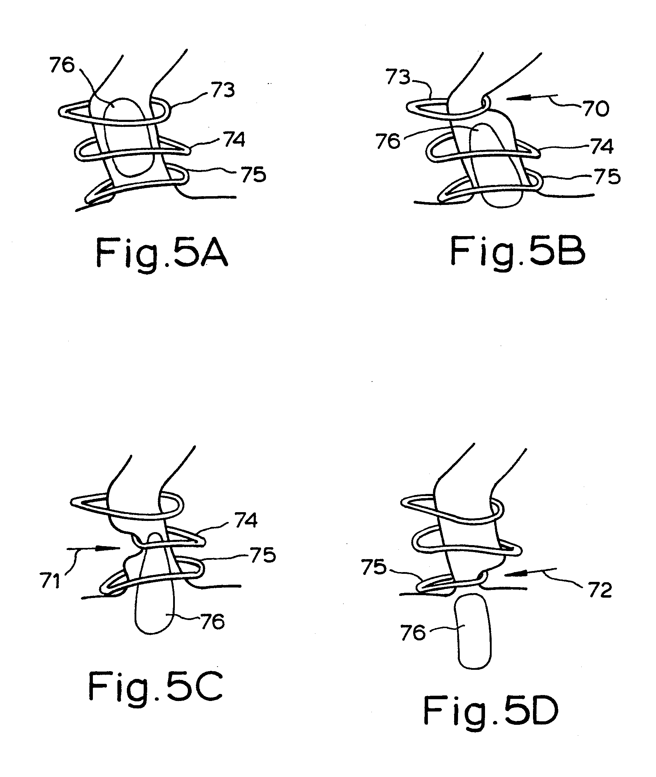

FIG. 5 further illustrates the reflexive action of the sphincter muscles after a foreign body enters the anal canal. Particularly depicted in FIG. 5 are the sequences of muscular actions to empty the anus of a foreign body whether it be a suppository or stool during defecation. The series of relaxations and contractions of the sphincter muscles causes a vermicular action. In FIG. 5A, all muscles denoted 73, 74 and 75 are relaxed prior to reflex as the foreign body 76 is introduced into the anal canal from the rectum. This is the same initial state when a suppository is introduced from the anal verge. In FIG. 5B, sphincter muscles 73 (profundus) contract at the upper end of the anal canal causing force 70 to propel the foreign body 76 downward to the more relaxed region 74. In FIG. 5C, the muscular region 74 (superficialis) contracts causing force 71 to propel the foreign body 76 downward to the more relaxed region 75. In FIG. 5D, foreign body 76 is expelled by the contraction of muscular region 75 (subcutaneous) causing force 72. The duration of time for the movement illustrated in FIGS. 5 A-D is approximately one or two seconds.

With reference to FIG. 2, the rigid suppository 110 is shown inserted in the anal canal 36. The suppository 110 differs from suppository 10 of FIG. 1 in not having a retrieval line through the distal end and in having a non-flat distal surface. In FIG 2, anal canal 36 is forced open by suppository 110 as it would be by any entering foreign body. While the suppository initially stretches the sphincter muscles, subcataneous 38, superficialis 40 and profundus 42, these muscles reflexedly tighten against the suppository as discussed above.

As shown in FIG. 2, proximal tip 112 of the suppository assumes a position inside rectum 44 above ano-rectal line 46. Shaft 116 occupies anal canal 36 and anal canal walls 48 thereby come in contact with peripheral walls 50 of shaft 116. As suppository 110 melts, the medication contained in the suppository material migrates directly into anal canal walls 48. Distal end 122 remains partially outside the body and partially in external anal orifice 52.

The bullet shape of proximal tip 112, positioned above ano-rectal line 46 enables suppository 110 to resist caudad force 54 (see FIG. 4) by leveraging against rectal walls 56. Likewise, the enlarged distal end 122, positioned in the external anal orifice 52 enables suppository 110 to resist the cephalad force 58 of the sphincter muscles by leveraging against the external anal orifice 52. As indicated above, it should be noted that distal end 122 of FIG. 2 differs in shape from the distal end 22 of FIG. 1 in having a curved bottom.

As suppository 110 melts, the opposing lateral forces 60, 62 and 64 (see FIG. 2) and 160, 162 and 164 (see FIG. 4) caused by sphincter subcutaneous 238, sphincter superficialis 240 and sphincter profundus 242, (FIG, 4) respectively, apply lateral pressure (the "scissor effect") against shaft 116. The resistance against expulsion of the suppository is greatly increased by the flexible communicating means 130 integrated therein which functions as an inner core for the suppository. Flexible communicating means 130 increases the structural strength of shaft 116 to resist being severed by the lateral forces. In the event that shaft 116 is broken by the scissor action of lateral forces, e.g. 60,62 and 64, or melts completely before the rest of suppository 110 has fully melted, inert flexible communicating means 130 anchored at ends 128 and 132 preserve the physical integrity of the remaining portions of suppository 110 to completely melt without being expelled by cephalad force 58 and caudad force 54. If the flexible communicating means 130 were not present and shaft 116 became severed, then cephalad force 58 would propel the proximal tip 112 into rectum 44 and caudad force 54 or vermicular action would eject distal end 122 outside the body. The flexible communicating means should be relatively inert during its presence in the anal canal. It should maintain its integrity during the period of time it is present in the anal canal. A dissolvable surgical cord, for example, will maintain its integrity while functioning as the flexible communicating means and therefore would be suitable, although it is capable of dissolving in the body after a prolonged period of time.

When suppository 110 has completely dispersed all medicine by melting, flexible communicating means 130 and enlarged ends 128 and 132 may be passed out of the body during the next natural defecation without trauma. Alternatively, an optional retrieving line may be incorporated into the suppository as in suppository 10 of FIG. 1 to facilitate removal of flexible communicating means 130 and any remaining, unmelted proximal tip 112 and distal end 122.

The non-dissolvable filament anchored within the suppository functions synergistically with the shape of the suppository when subjected to the dynamic stresses of the anal canal. Without the presence of the non-dissolvable filament, a suppository of the same shape will not function successfully and remain intact in the anal canal during the time required for treatment. This is demonstrated below in Example 1.

### Example 1

The suppositories used in this Example have the shape and dimensions of FIG. 1, except that the flexible communicating means or filament 30 is not embedded therein. The anal suppository was inserted into an adult patient. After about 1 minute, the distal or external end was expelled with about three-quarters of the length of the shaft. The shaft portion was slightly melted, i.e. the surface was softened. The top or proximal end and remaining portion of the shaft apparently slipped upward into the rectum. This experiment was repeated twenty times with various patients and each time resulted in the severing and expulsion of the distal end and shaft portion from the body of patients. The explanation of why such suppositories were severed was developed after a careful analysis of the physiology of the anal canal.

Firstly, the point on the shaft where severing occurred corresponds to the point in the anal canal where maximum pressure is exerted, about 2 cm.from the anal verge. Such pressure accelerates melting of the shaft. The second factor involved the three sphincter muscles which act upon the anal canal. These are, in order from the outer end or anal verge and with reference to FIG. 3, the sphincter subcutaneous 38, the sphincter superficialis 40 and the sphincter profundus 42. Two of these muscles namely the sphincter subcutaneous and the sphincter profundus, exert force upon the anal canal in one direction and the third muscle, the sphincter superficialis, exerts force upon the anal canal in the opposite direction. The result is that the anal canal, at rest, is closed and constricted in an "S" shape. At the completion of defecation, the superficialis muscle contracts from either side toward the centre functioning like two blades of a scissor cutting the remaining stool. The constricting action of the profundus muscle pushes the upper portion of the stool back into the rectum while the subcutaneous muscle pushes the lower portion of the stool out of the anus to the outside. The effect of this activity on the suppository of this example, is that a lateral, cutting force referred to as the "scissor effect" or "closing reflex" is applied which severs the suppository. The functioning of the sphincter muscles is more fully described in: "The Riddle of the Sphincters", Marvin Schuster, M.D., Gastroenterology, Vol. 69, No. 1, p 249 (1975); "A New Concept of the Anatomy of the Anal Sphincter Mechanism and the Physiology of Defecation", Ahmed Shafik, Investigative Urology, Vol. 12, No. 5, p 412 (1975); Duthie, H. L. et al, "Contribution of the External Anal Sphincter to the Pressure Zone in the anal canal", Gut, Vol. 6, pp 64-68, (1965); and Goligher, J. C. et al., "Surgery of the Anus, Rectum and Colon", Baillicre Tindall, London, Third Ed. (1975).

## Example 2

A moulded suppository in accordance with the present invention as illustrated in FIG. 1, including the flexible communicating means 30, was used for this experiment. The flexible communicating means was a string bearing a knot at each end to function as the anchors when embedded in the suppository. The string was dipped in paraffin to impart stiffness and improve handling of the string. Each suppository contained two grains of benzocaine as a topical anesthetic in cocoa butter and 10% bees wax. Twenty of these suppositories were introduced into patients and all remained in place until the axial shaft portion was completely dissolved. The residual string, remaining proximal tip and distal portions were mostly clean of the medication when removed by the patients pulling the string tail. Examination anoscopically indicated that the upper spheroid of the suppository had bathed the dentate region and the shaft bathed and anal canal, while the distal spheroid bathed the external anal and peri-anal area.

## Example 3

Some of the dimensions of the suppository according to the present invention are more important than others. This example illustrates that certain variations in the length of the suppository having the general shape of FIG. 1 may render the suppository vulnerable to expulsion from the anal canal prior to dissolution. A suppository was prepared having the shape illustrated in FIG. 1, but without the flexible communicating means within and having a longitudinal overall length of 4 cm, the proximal tip being 1 cm, the axial shaft 2 cm and the distal end 1 cm. Twenty suppositories were prepared as aforesaid by hand rolling from suppositories sold by Wyeth Laboratories, Inc. of Philadelphia, Pennsylvania, U.S.A., under the trade name "Wyanoids HC". These suppositories were inserted into twenty different patients, ten with anal pathology and ten without. Each and every suppository was immediately expelled from the patient without melting.

As shown in Example 2, the suppositories in accordance with the present invention are not expelled. The suppositories of Example 2 have an overall length of 5.1 cm and the length of the axial shaft is 2.8 cm. It is believed that each of the suppositories used in the present example was expelled because its length, particularly that of the axial shaft portion, was too short and, when the suppository was inserted, the proximal tip was not in a position sufficiently abutting the anorectal wall to provide the suppository with resistance from the downward caudad force of the sphincter muscles. The average length of the relaxed anal canal of an adult is about 3.2 cm. The length of the anal canal shrinks only slightly with muscle contraction. In order for the suppository to function properly, the length of the anal canal should be coextensive with the axial shaft of the suppository or with the axial shaft plus a small portion of the proximal and distal ends facing or adjacent the axial shaft. Since the cross-sectional diameter of the proximal and distal ends increases only gradually from the axial shaft, a portion of the ends immediately adjacent the axial shaft may reside within the anal canal during treatment without causing discomfort yet still maintain resistance to the caudad and cephalad forces. From this analysis, it is clear that the length of the axial shaft will depend, in part, on the shape or degree of increase of the cross-sectional diameter of the ends relative to that of the axial shaft.

The cross-sectional dimensions of the suppository have a much wider tolerance than the length dimensions. One reason for this is that the anal canal normally adjusts its cross-sectional dimensions and flexes to cause a snug fit about any foreign body including the suppository. However, the length of the anal canal does not vary significantly by muscular action, hence, the importance of a length of commensurate with the anal canal. Generally, the diameter of the axial shaft of the suppository should be at least 5 mm. to provide sufficient volume to carry an effective amount of medication and to have structural integrity during handling.

The suppositories in accordance with the present invention may be considered solid dosage forms for transmitting medication to the region of the anal canal. Typical non-prescription drugs or therapeutic substances which may be included in suppositories include: astringents, such as bismuth salts, zinc oxides or tannic acid, local anesthetics, such as benzocain, dibucaine, disperodon or tetracaine; antiprurities, such as menthol or phenol; local anesthetics; antiseptics, such as benzethonium chloride, boric acid, phenylmercuric citrate, hexachlorophene or phenol; emollients, such as cod liver oil, lanolin, castor oil or petrolatum; and vasoconstrictors, such as ephedrine sulphate. Typical prescription drugs which are utilized in anal suppositories include anti-inflammatory drugs, antibiotics and steroids. The concentration of the drug or therapeutic substance which is dispersed by the suppository is a function of the concentration of the drug in the suppository, as well as its solubility or suspension in the base or melting material and rate of dissolution. The base composition of the suppository normally dissolves because it has a melting point at or below the temperature of the anal canal. Also the dissolution of the base material included therein, as well as by body fluids within the anal canal. The base composition for the suppository must therefore be chosen with consideration given to the medicaments included therein. The most popular base materials in suppositories are theobroma oil or cacao butter (cocoa butter). The melting point of theobroma oil, for example, is from 30 to 35°C (from 86 to 95°F). The treatment times to be provided by the suppositories in accordance with the present invention may vary from one minute to many hours depending upon the type of treatment to be provided. The rates of suppository melting and distribution of medication may be controlled, for example, by using a suppository body of heterogeneous composition. A good discussion of the materials utilized in anal suppositories appears in Remington's Pharmaceutical Sciences, 15th Edition, 1975, published by Mack Publishing Company at pages 1546-1553. Treatment time by the suppositories in accordance with the present invention may be further extended by utilizing a flexible communicating means capable of being impregnated with medication such that medication continues to be provided to the anal canal after the axial shaft has dissolved.

The suppositories in accordance with the present invention provide therapy not provided by known suppositories. For example, spasms may be relaxed to a degree only heretofore obtained by injection of local anesthetic. In comparison with conventional suppositories, the suppositories in accordance with the present invention attain more successful treatment of anal disorders even when containing less active therapeutic ingredients.

Example 4

The following case studies illustrate successful treatment utilizing the suppository in accordance with the present invention:

Patient A suffering from intense anal pain and burning received no relief from repeated use of the commercially available "Wyanoid HC" rectal suppository indicated as providing analegesic, anti-inflammatory, astringent and soothing therapy. Examination of the patient was not possible due to the pain. An anal suppository in accordance with FIG. 1 made from Wyanoid HC suppository material with added benzocaine was inserted into the patient. Within minutes, the patient reported excellent relief and was able to undergo examination.

Patient B suffering from anal discomfort due to prior surgery obtained no relief from repeated use of "Anusol HC" rectal suppositories as manufactured by Parke-Davis division of Warner-Lambert Co. The Anusol HC suppository is described as containing bismuth subgallate 2.25%, bismuth resorcin compound 1.75%, hydrocordisone 10 mg, benzyl benzoate, 1.2%, peruvian balsam 1.8 %, zinc oxide 11.0%, in a bland hydrogenated vegetable oil base. Examination indicated that healing of the lower third of the anal canal was incomplete and appeared unaffected and unbathed by the Anusol suppository. An anal suppository in accordance with FIG. 1 made from Anusol HC suppository material with 3 grains of benzocaine provided complete relief to the patient within four minutes after insertion.

Patient C complained of severe anal pain. Examination revealed an ulceration following prior-treatment of intra-anal condyloma. An anal suppository according to FIG. 1 made from Wyanoid HC suppository material was inserted into the patient. Complete relief was reported by the patient.

Patient D complained of anal pain especially following bowel movement and complained of bowel urgency. Examination revealed a high post-operative anal tear with multiple painful fissures. The patient had repeatedly used Anusol rectal suppositories without relief. An anal suppository according to FIG. 1 made from Anusol suppository material with 3 grains benzocaine was inserted. The patient reported complete relief in about five minutes.

Patient E complained of having symptoms of anusitis for more than one year and had been using Preparation H without relief. An anal suppository according to FIG..1 made from Anusol HC with 3 grains benzocaine was inserted. The patient reported complete relief in minutes.

Additional patients complaining of anal pain or burning were treated with an anal suppository, according to FIG. 1, made from either available Anusol suppository material or available Wyanoid HC suppository material without the addition of any other medication. After this treatment, each patient reported improved to complete relief. These patients had previously used one or both of the commercially available Anusol or Wyanoid HC suppositories without obtaining relief.

CLAIMS:

1.      An anal suppository characterised in that it is capable of being inserted and retained in the anal canal and melting or dissolving therein while dispersing medication to the walls of the anal canal, the suppository comprising a composition which melts or dissolves in the anal canal and having a configuration which comprises an enlarged proximal tip (12) extending from one end of a cylindrically-shaped axial shaft (16) and an enlarged distal portion (22) extending from the opposite end of the axial shaft wherein a flexible communicating means (30) composed of a flexible and atraumatic composition, relatively inert to the environment of the anal canal is embedded in the central core of the suppository from the proximal tip through the axial shaft to the distal portion and anchored in the proximal and distal portions.

2.      A suppository as claimed in claim 1 wherein the axial shaft extends the full length of the anal canal when inserted therein and the proximal tip and distal portion have a cross-section sufficiently large to prevent expulsion of the suppository from the anal canal yet sufficiently small to enable insertion and not be traumatic.

3.      A suppository as claimed in claim 1 or claim 2 wherein the proximal tip is bullet-shaped.

4.      A suppository as claimed in any of claims 1 to 3 wherein the distal end is mushroom-shaped.

5.      A suppository as claimed in any of claims 1 to 4 wherein the flexible communicating means comprises enlargements at its ends to function as anchors and to improve cohesion in the proximal tip and distal portion.

6.      A suppository as claimed in claim 5 wherein the flexible communicating means is an extruded filament having enlargements at its ends.

7.      A suppository as claimed in claim 5 wherein the flexible communicating means is a string or cord having knots at its ends.

8.      A suppository as claimed in any of claims 1 to 7 wherein the flexible communicating means extends through the distal end surface to provide a tail or retrieval line.

Fig.1

Fig.2

Fig.3

Fig.4

Fig.5A

Fig.5B

Fig.5C

Fig.5D